# EUROPEAN PATENT APPLICATION

(11) **EP 4 273 219 A1**
(43) Date of publication of application: **08.11.2023**
(21) Application number: 22171879.4
(22) Date of filing: 05.05.2022
(51) Int. Cl.: C12M 3/06, C12M 1/12, C12M 1/42

(54) **MICROFLUIDIC DEVICE**

(71) Applicant: Eberhard Karls Universität Tübingen, Medizinische Fakultät, 72074 Tübingen (DE)
(72) Inventor: Loskill, Peter, 72070 Tübingen (DE); Schlünder, Katharina, 72072 Tübingen (DE)
(74) Representative: Witte, Weller & Partner Patentanwälte mbB

(57) **Abstract**

The present invention relates to a microfluidic device, a microfluidic system comprising said microfluidic device, and to a method of culturing biological material.

## Description

The present invention relates to a microfluidic device, a microfluidic system comprising said microfluidic device, and to a method of culturing biological material.

### FIELD OF THE INVENTION

The present invention generally relates to the field of cell or molecular biology, especially to the field of microfluidics. More particularly, it concerns a biochip using microfluidic technology.

### BACKGROUND OF THE INVENTION

In molecular biology, biochips are engineered substrates ("miniaturized laboratories") that can host large numbers of simultaneous biochemical reactions. One of the goals of biochip technology is to efficiently screen large numbers of biological analytes, with potential applications ranging from disease diagnosis to detection of bioterrorism agents.

An organ-on-a-chip (OOC) is a multi-channel 3-D microfluidic cell culture, integrated circuit (chip) that simulates the activities, mechanics and physiological response of a tissue or an entire organ or an organ system, a type of artificial organ. It constitutes the subject matter of significant biomedical engineering research. It is a fit for purpose fabricated microfluidic-based device, containing living engineered organ substructures in a controlled micro- or nanoenvironment, that recapitulate aspects of the dynamics, functionality and (patho)physiological response of an organ *in vivo,* in real-time monitoring mode. The convergence of labs-on-chips (LOCs) and cell biology has permitted the study of human physiology in an organ-specific context, introducing a novel model of *in vitro* multicellular human organisms.

Although multiple publications claim to have translated organ functions onto this interface, the movement towards this microfluidic application is still in its infancy. Organs-on-chips will vary in design and approach between different researchers. As such, validation and optimization of these systems will likely be a long process. Organs that have been simulated by microfluidic devices include brain, lung, heart, kidney, liver, prostate, vessel(artery), skin, bone, cartilage and more.

Nevertheless, building valid artificial organs requires not only a precise cellular manipulation, but a detailed understanding of the human body's fundamental intricate response to any event. A common concern with organs-on-chips lies in the isolation of organs during testing. The body is a complex network of physiological processes, making it challenging to simulate a single organ.

Rennert et al. (2015), A microfluidically perfused three dimensional human liver model, Biomaterials, Vol. 71, p. 119-131, disclose a biochip intended to simulate and analyze *in vitro* the functioning of the human liver. The authors report the establishment of a three-dimensional liver organoid embedded in microfluidically-supported biochips, which is supposed to be structurally inspired by the morphology of the liver.

Rogal et al. (2020), WAT-on-a-chip integrating human mature white adipocytes for mechanistic research and pharmaceutical applications, Scientific Reports, Vol. 10, p. 1-12, describe the development of an organ-on-a-chip that integrates functional mature human white adipocytes. The known chip is a multilayer device that features tissue chambers tailored specifically for the maintenance of 3D tissues based on human primary adipocytes, with supporting nourishment provided through perfused media channels.

Hori et al. (2019), Compact fluidic system for functional assessment of pancreatic islets, Biomedical Microdevices, Vol. 21, p. 1-9, disclose a compact fluidic system that is supposed to enable assessment of pancreatic islet functionality ex *vivo* for efficient islet transplantation. The known fluidic system includes a micromesh sheet-embedded chip. Islets can be loaded on the micromesh sheet and observed by microscopy. The authors report that islets on the mesh sheet mainly remained in place during perfusion and did not get damaged by hydraulic pressure because of high porosity of the micromesh sheet. The known fluidic system was assembled with a sample fraction chip of polydimethylsiloxane. The chip includes a channel and columns, both having surfaces that were super-hydrophilized so that solutions could flow within the chip by gravity.

Nourmohammadzadeh et al. (2013), Microfluidic array with integrated oxygenation control for real-time live-cell imaging: effect of hypoxia on physiology of microencapsulated pancreatic islets, Analytical Chemistry, Vol. 85, p. 11240-11249, disclose a microfluidic islet array based on a hydrodynamic trapping principle. According to the authors the array can be used for lab-on-a-chip studies with live-cell multiparametric imaging which supposed to allow understanding of physiological and pathophysiological changes of microencapsulated islets under hypoxic conditions.

Zbinden et al. (2020), Non-invasive marker-independent high content analysis of a microphysiological human pancreas-on-a-chip model, Matrix Biology, Vol. 85-86, P. 205-220, disclose an endocrine pancreas-on-a-chip model based on a tailored microfluidic platform, which enables self-guided trapping of single human pseudo-islets.

The biochips currently used in the state of the art use auxiliary structures and still do not allow *in vitro* modeling of the complex microenvironment of biological organs, such as pancreatic islets. In particular, the known biochips do not allow *in situ* analyses of the artificial organs, especially because the artificial organs cannot be localized or immobilized in a targeted manner at definite locations in the chip and simultaneously cultivated and analyzed under physiological conditions. It is therefore the object of the present invention to provide a device in which the disadvantages prevailing in the prior art are avoided or at least reduced. In particular, a microfluidic device is to be provided which brings artificial organ structures into a physiological environment and enables in situ analysis thereof.

The present invention satisfied these and other needs.

### SUMMARY OF THE INVENTION

The object underlying the invention is solved by the provision of a microfluidic device, comprising a first microfluidic channel, a second microfluidic channel and a porous membrane, wherein at least a section of said first and second microfluidic channels is separated by said porous membrane, characterized in that said porous membrane comprises trapping structures at predefined positions configured to immobilize biological material.

The term "microfluidic" as used herein relates to components where moving fluid is constrained in or directed through one or more channels wherein one or more dimensions are 1 mm or smaller (microscale). Microfluidic channels may be larger than microscale in one or more directions, though the channel (s) will be on the microscale in at least one direction. In some instances, the geometry of a microfluidic channel may be configured to control the fluid flow rate through the channel (e.g. increase channel height to reduce shear). Microfluidic channels can be formed of various geometries to facilitate a wide range of flow rates through the channels and the integration and three-dimensional setup of biological tissues.

"Channels" are pathways (whether straight, curved, single, multiple, in a network, etc.) through a medium (e.g., polymethylmethacrylate) that allow for movement of fluids or liquids and gasses. Channels thus can connect other components, i.e., keep components "in communication" and more particularly, "in fluidic communication" and still more particularly, "in liquid communication". Such components include, but are not limited to, liquid-inlet openings and gas vents. "Microfluidic channels" are channels with dimensions less than 1 millimeter and greater than 1 micrometer.

According to the invention a "porous membrane" refers to a structure made of a biocompatible material with pores large enough to only permit exchange of gases and/or small chemicals, or large enough to permit migration and transchannel passage of large proteins, as well as whole living cells and/or portions thereof. Due to these properties it is also referred to a semi-permeable membrane. The membrane may be porous, flexible, elastic, flatly or a combination thereof.

According to the invention, the first and second microfluidic channels are, at least at a common section thereof, "separated" by the porous membrane. That is, at the section a first surface of the porous membrane forms an interface with the lumen of the first microfluidic channel, and the second surface on the other side of the porous membrane forms an interface with the lumen of the second microfluidic channel. In other words, at said section the first surface of the porous membrane bounds the lumen of the first microfluidic channel, and the second surface of the porous membrane bounds the lumen of the second microfluidic channel. For this reason, in said section, the first and second microfluidic channels run essentially parallel and adjacent to each other, connected by the porous membrane. Beyond said section, the courses of the first and second microfluidic channels may differ.

According to the invention, a "trapping structure" refers to an arrangement at a specific position in the porous membrane to which biological material can "immobilize", i.e. attach and adhere in a directed manner on a long-term or permanent basis. The trapping structures are located at "predefined positions" on or in the porous membran, which means that the locations of the trapping structures are not randomly distributed or randomly created, but are positioned by the skilled person at well-defined and previously determined locations on or in the membrane.

The microfluidic device according to the invention allows, in an advantageous manner, the three-dimensional tissue culturing *in vitro* by modelling complex physiological microenvironments such as those found in or around the human Langerhans or pancreatic islets. It provides a microphysiological system (MPS), which enables self-guided immobilization of biological material, such as single cell spheroids (e.g. pseudo pancreatic islets), at defined locations to enable *in situ* analysis on-chip. The biological material is not separated by auxiliary structures like in the devices known in the art but only by the surrounding structures, such as extracellular matrix- (ECM) like hydrogel. The microfluidic device according to the invention is, therefore, a promising tool supporting 3-D tissue culture of physiological models and the integration of further relevant tissue components, while providing an *in vitro* platform for non-invasive analysis of cell (patho-) physiology and response to drug treatment.

In an embodiment of the invention said trapping structures are through holes in said porous membrane, preferably comprising a mean diameter of approx. 30 - 70 µm, preferably approx. 40 - 60 µm, further preferably approx. 50 µm.

This measure has the advantage that such structures are provided in the membrane on which the biological material can "sit" when it flows through the first channel. In this case, the loading of the trapping structures with the biological material can be achieved by a suitable adjustment of the pressure conditions in the channels. After the biological material has been introduced into the first microfluidic channel, the pressure along the first microfluidic channel is thereby adjusted so that it is lower than the pressure within the first microfluidic channel exerted on the porous membrane in the direction of the second microfluidic channel. The biological material thereby settles in the hole-like structures and is immobilized there. However, the dimensions can be adjusted, depending on the actual needs. For examples larger diameters, e.g. of approx. 100 µm can be provided to immobilized even larger biological material. Depending on the nature and material of the porous membrane, the through holes can be made by means known to the skilled person, such as drilling, punching, cutting, in particular by using a UV laser cutter.

In another embodiment of the microfluidic device said first and second microfluidic channels comprise an average mean diameter of approx. 400 - 600 µm, preferably approx. 500 µm.

This measure advantageously creates the constructive prerequisites for ensuring that the channels can be properly loaded with biological material, such as cell spheroids with average diameters of approx. 150 µm, and liquids, such as cell culture solutions, in compliance with the laws of microfluidics. However, the dimensions can be adjusted, depending on the actual needs.

In yet another embodiment of the microfluidic device according to the invention said first microfluidic channel comprises, at a first end, an inlet opening. In another embodiment said first microfluidic channel comprises, at a second end, a dead end structure.

This measure has the advantage that the biological material, possibly dissolved in a liquid, as well as other components such as hydrogel, can be introduced into the device or the first microfluidic channel via the inlet opening. The provision of a dead end structure, which might be a terminal end of the channel in the support or carrier material surrounding the channel, has the advantage that the biological material and other material introduced into the first microfluidic channel remain in the latter and are not flushed out again.

In another embodiment of the microfluidic device of the invention said second microfluidic channel comprises, at a first end, an inlet opening and, at a second end, an outlet opening.

This measure allows fluid or liquid to be introduced via the inlet opening into the second microfluidic channel, e.g. cell culture medium, and to be discharged from the second microfluidic channel via the outlet opening. In this way, the biological material, especially that which is immobilized in the trapping structures, can be continuously supplied with fresh medium via a fluid flow. Furthermore, test substances, such as pharmacologically active substances, can be introduced into the second channel, incubated there for any desired and adjustable time, and then rinsed out again via the outlet opening.

In another embodiment of the microfluidic device according to the invention said first microfluidic channel is provided in a first plate and said second microfluidic channel is provided in a second plate.

The first and/or second plate can be a carrier or support made of any material in which the first and/or second microfluidic channel can be provided or incorporated, such as Plexiglas, silicone or comparable material. The provision of carrier plates makes the microfluidic device manageable and easy to use in practice.

In yet another embodiment of the microfluidic device said first and/or second plates are transparent.

This measure creates the constructive prerequisites for the microfluidic device and the biological material contained therein to be microscopically accessible and investigable and, in particular, for emitted light or fluorescence signals to be detected with suitable detectors.

According to an embodiment of the invention said first and second plates of the microfluidic device comprise a material selected from the group consisting of: polymethylmethacrylate (PMMA), polydimethylsiloxane (PDMS), polycarbonate (PC), thermoplastic elastomers (TPE), glass, cyclic olefin copolymer (COC), etc.

This measure has the advantage of using materials into which the microfluidic channels can be incorporated with known and simple methods in the desired dimensions, which are light and at the same time ensure stability, and can be designed to be transparent.

In still another embodiment of the microfluidic device according to the invention said porous membrane has a thickness of approx. 10 - 30 µm, preferably of approx. 20 - 25 µm, further preferably of approx. 22 µm.

According to the inventors' findings, the dimensions given are optimal for the porous membrane to perform the tasks of the invention and to fit well into the device.

In another embodiment of the microfluidic device according to the invention said porous membrane comprises pores comprising a mean diameter of approx. 1-10 µm, preferably approx. 1.5-8 µm, further preferably approx. 2-3 µm.

According to the inventors, this pore size creates semi-permeability. It ensures that only small molecules such as gases, metabolic products, active ingredients such as insulin, etc. can pass through the membrane, but not biological material such as cells.

According to another embodiment of the microfluidic device of the invention said porous membrane comprises polycarbonate (PC).

In this embodiment material for the manufacturing of the porous membrane is used which meets the requirements needed for a proper functioning of the microfluidic device. However, alternative materials can be used, such as polyethylene terephthalate (PET), polydimethylsiloxane (PDMS), vitrified hydrogel, electropun polymers etc.

In another embodiment the microfluidic device according to the invention comprises a top plate adjacent to said second plate, and, in another embodiment, a bottom plate adjacent to said first plate.

The provision of a bottom and/or top end plate gives the microfluidic device the stability required for robust laboratory use. Holes can be provided in the top plate to provide access to and connect with the inlet and outlet ports of the first and/or second microfluidic channel, respectively.

The top and/or bottom plates may, in embodiments of the invention, also be kept transparent and made of a material selected from polymethylmethacrylate (PMMA), polydimethylsiloxane (PDMS), polycarbonate (PC), thermoplastic elastomers (TPE), glass, cyclic olefin copolymer (COC), etc. In this respect, what was previously said for the first and second plates applies accordingly to the top and bottom plates.

In still another embodiment of the microfluidic device of the invention said bottom plate comprises a sensor.

While any kind of contactless sensors can be comprised by the bottom plate the provision of an oxygen sensor is exemplified. The oxygen sensor can be realized by an oxygen indicator dye. The latter can be spotted onto the bottom plate.

In another embodiment of the microfluidic device said biological material comprises cellular aggregates, preferably cell spheroids, preferably comprising insulin-producing cells, further preferably beta cells.

With this measure, the device according to the invention is further converted into a functional biosensor or microphysiological system (MPS), respectively. Cellular aggregates are accumulations of biological cells in a functional composite. An example of a cellular aggregate is a cell spheroid. Spheroids are a type of three-dimensional cell modeling that better simulate a live cell's environmental conditions compared to a two-dimensional cell model, specifically with the reactions between cells and the reactions between cells and the extracellular matrix (ECM) or the ECM-like hydrogel, respectively. Typically, a cell spheroid has a mean diameter of approx. 120-300 µm.An example of a cell spheroid is a pseudo pancreatic islet, which, in such embodiment, results in the provision of a microphysiological pancreas-on-chip platform. Therefore, in another embodiment of the invention said biological material comprises insulin-producing cells, preferably beta cells.

Another subject-matter of the invention relates to a microfluidic system comprising the microfluidic device according to the invention and a fluid source in fluidic communication with said second channel.

The features, characteristics and advantages mentioned for the microfluidic device apply likewise to the microfluidic system according to the invention.

Another subject-matter of the invention relates to a method of culturing biological material, comprising: a) providing the microfluidic device according to the invention; b) introducing biological material into said first microfluidic channel; c) introducing culture medium into said second microfluidic channel, and d) culturing said biological material under conditions allowing a physiological functioning of said biological material.

The features, characteristics and advantages mentioned for the microfluidic device apply likewise to the method according to the invention.

Against this background, in an embodiment of the method according to the invention said biological material comprises cellular aggregates, preferably cell spheroids, preferably comprising insulin-producing cells, further preferably beta cells.

In another embodiment of the method according to the invention in step (b) further material is introduced into said first microfluidic channel. Preferably, said further material comprises biological cells and/or hydrogel, preferably ECM-like hydrogel.

This measure can advantageously create an environment for the biological material or cell spheroids that largely corresponds to the natural environment. Interactions of the biological cells with each other and/or with the ECM can take place, so that in particular the functionality of a pancreas can be simulated and investigated.

In another embodiment of the method of the invention in step (b) the introduction of said biological material into said first microfluidic channel is realized via the application of a hydrostatic-pressure driven flow.

By this measure physical forces are used in an advantageous manner to load the microfluidic device and to bring the biological material in the desired place. In this embodiment the pressure conditions in the first microfluidic channel are adjusted so that the pressure along the first microfluidic channel is lower than the pressure within the first microfluidic channel exerted on the porous membrane in the direction of the second microfluidic channel. The biological material thereby settles in the hole-like trapping structures and is immobilized there. The adjustment can be realized by establishing an appropriate height difference between the inlet opening of the first microchannel and the outlet opening of the second microchannel.

In another embodiment of the method according to the invention the latter comprises the following further step e'), within which the biological material is examined, preferably visually, further preferably microscopically and/or spectroscopically.

In still another embodiment the method according to the invention comprises the following further step c') within which said culture medium is exited from said second microfluidic channel, and, preferably said exited culture medium is examined, further preferably for compounds secreted by said biological material.

It is to be understood that the before-mentioned features and those to be mentioned in the following cannot only be used in the combination indicated in the respective case, but also in other combinations or in an isolated manner without departing from the scope of the invention.

The invention is now described and explained in further detail by referring to the following non-limiting examples and drawings.

### BRIEF DESCRIPTION OF THE DRAWINGS

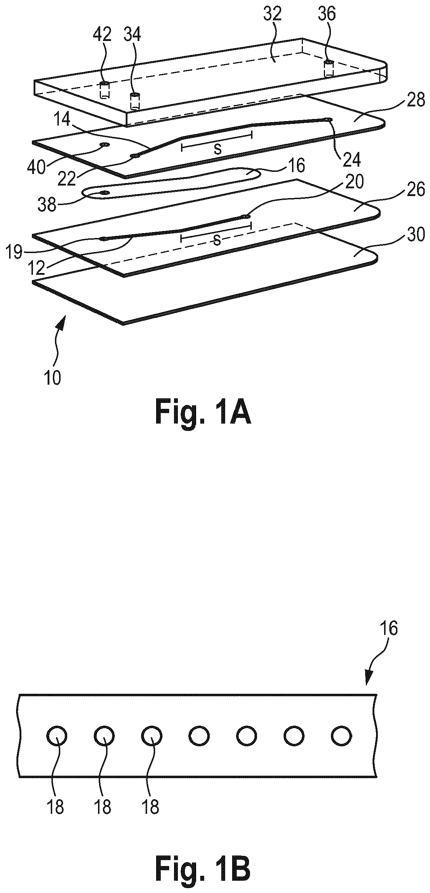

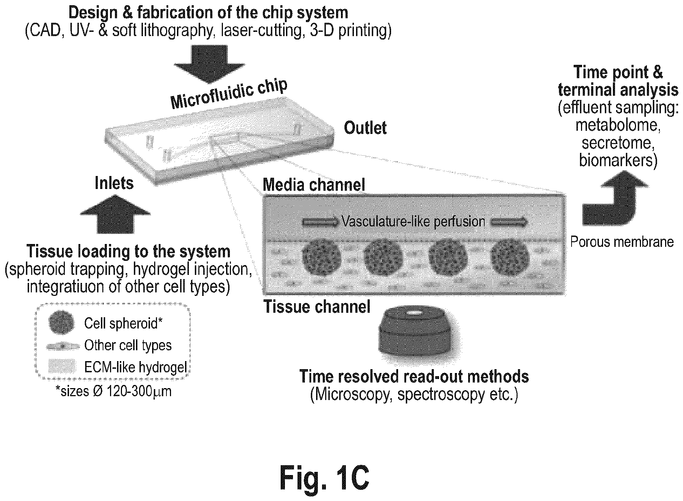

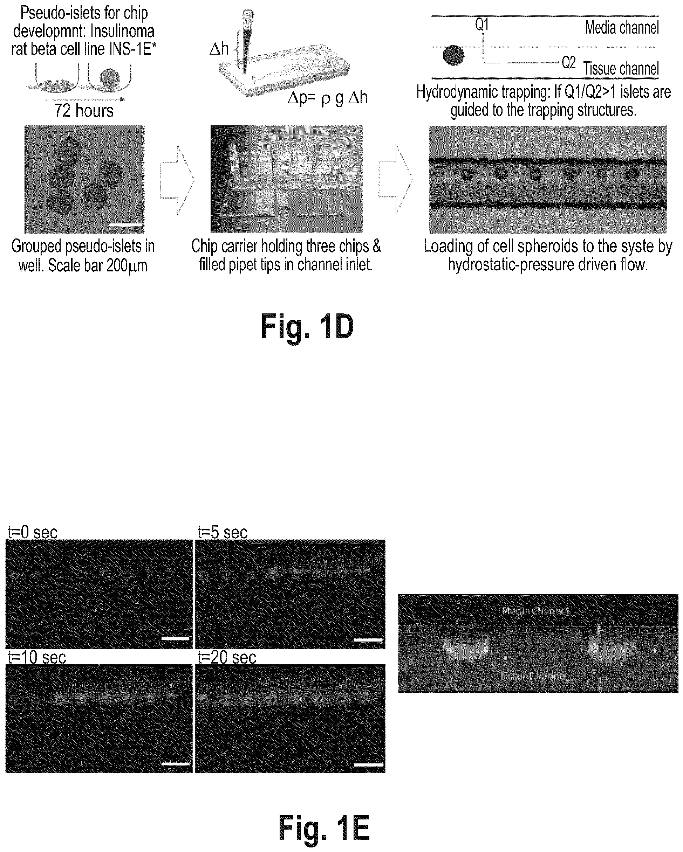

- Fig. 1:: Microfluidic chip concept and design. (A) Explosive view of an embodiment of the microfluidic device according to the invention. (B) Schematic illustration of the trapping structures comprised by the porous membrane. (C) Schematic of chip concept, fabrication, tissue loading & analysis. (D) Loading concept of the beta-cell pseudo-islets to the tissue chamber. Pseudo-islets are grouped and injected to the pipet tip located in the inlet of the tissue channel. Loading is achieved by hydrostaticpressure driven flow resulting from the height difference of the tissue inlet compared to the outlets of the media channel. Pseudo-islets are guided to the trapping positions along the membrane due to flow differences. (E) Left: Subsequent filling of the tissue chamber with FibriCol hydrogel also using hydrostatic pressure-driven flow. Invitrogen FluoSpheres added to the hydrogel for visualization. Scale bar 500 µm. Right: Maximum intensity projection of z-stack of chip system on Day 1 of on-chip culture. Fluorescent microspheres in the hydrogel and FDA/PI stained pseudo-islets.

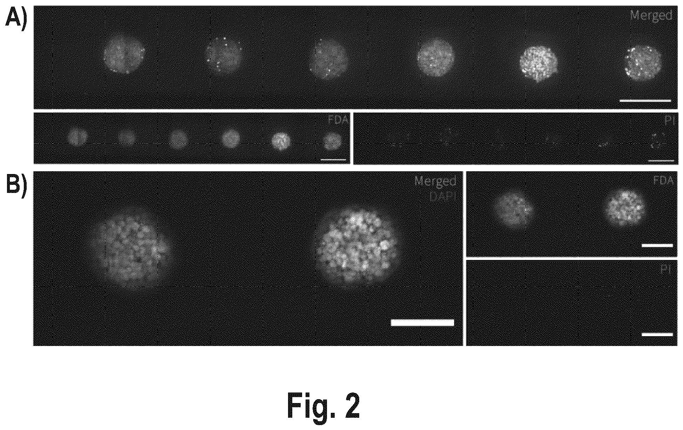

- Fig. 2:: Viability on-chip. Pseudo islets on-chip were stained with FDA/PI and DAPI. (A) Maximum intensity projection of a tile-scan and z-stack of the whole channel. Imaged on Axio Z.1 Cell Observer Spinning Disk (Zeiss). Scale bar 200 µm.(B) Two pseudo-islets on chip imaged on laser scanning confocal microscope LSM 710 (Zeiss). Scale bar 200 µm.

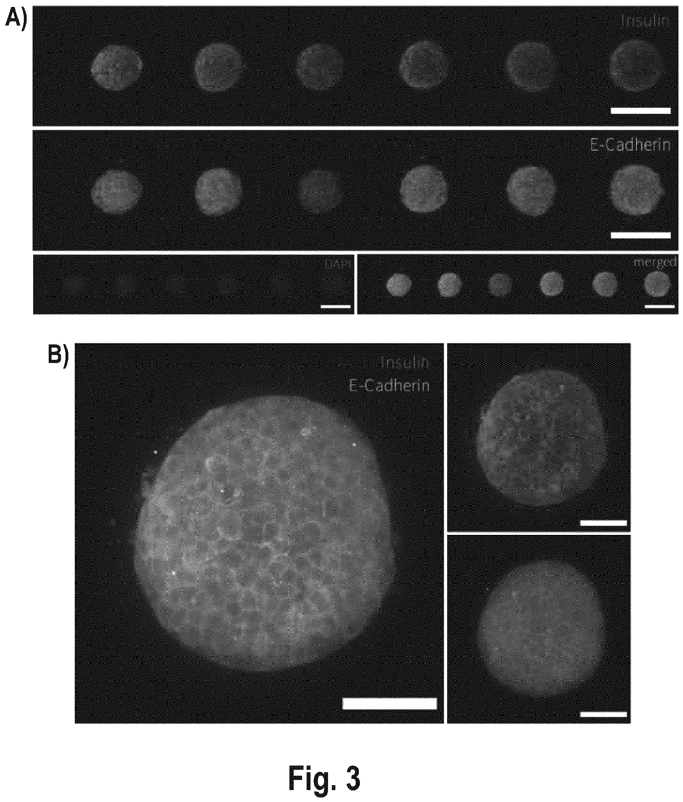

- Fig. 3:: Insulin and e-cadherin expression on-chip. (A) Maximum intensity projection of a tile scan and z-stack of the whole channel of a chip. Imaged on Axio Z.1 Cell Observer Spinning Disk (Zeiss). Scale bar 200 µm.(B) Maximum intensity projection of one pseudo-islet on-chip. Imaged on Axio Z.1 Cell Observer Spinning Disk (Zeiss). Scale bar 200 µm.

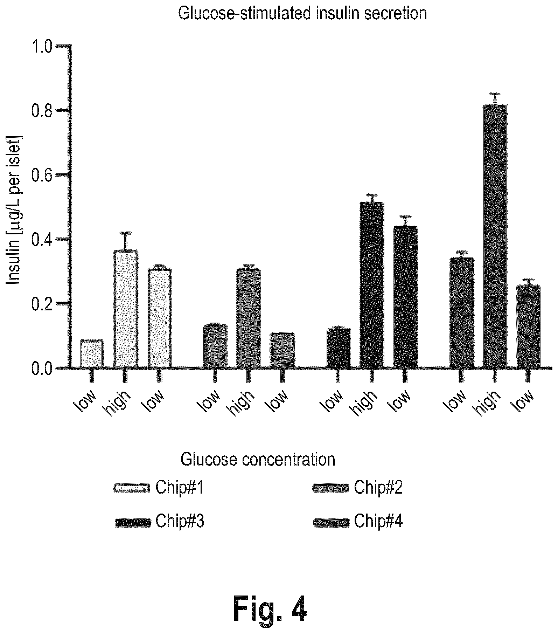

- Fig. 4:: Glucose-stimulated insulin secretion. (A) Insulin secretion of pseudo-islets on-chip in response to glucose stimulation with low (3.3 mM) and high (16.7 mM) glucose levels. Effluent was collected for each of the glucose conditions (low-high-low) after 60 min of perfusion from the outlet of the media channel. (B) n=5 chips, one-way ANOVA, *p < 0.05, **p < 0.005, ***p < 0.0005.

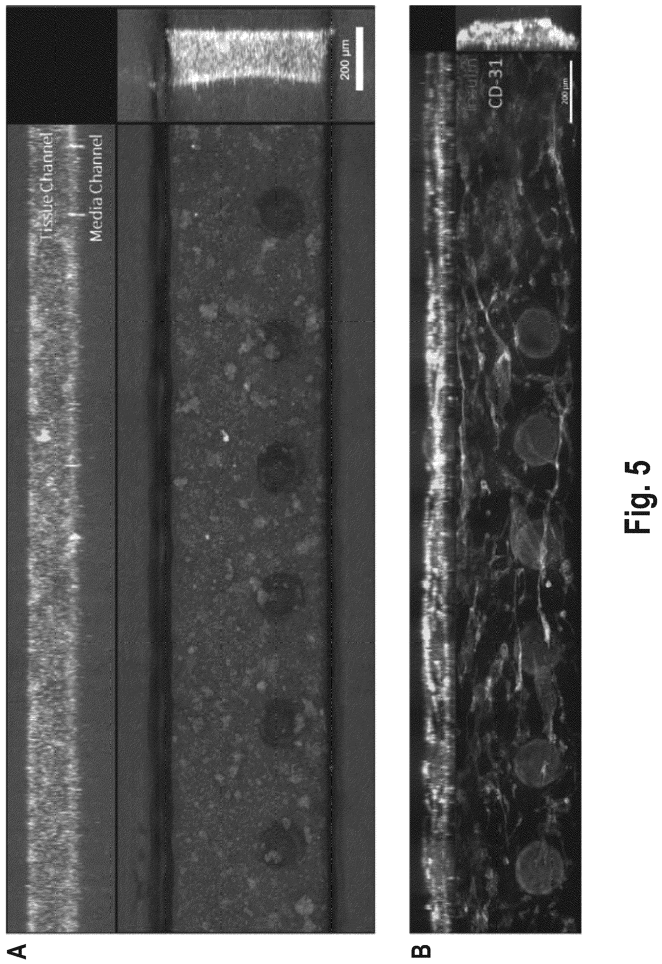

- Fig. 5:: Integration of tissue-relevant cell types. Endothelial cells were suspended in the hydrogel and loaded to the tissue chamber of the chip system. (A) Orthogonal view and maximum intensity projection of the imaged chip system 1 h after hydrogel & cell injection. FluoSpheres & CellTracker Deep Red (Invitrogen^{™}) for visualization of hydrogel and endothelial cells, respectively. Scale bar 200 µm. Imaged on LSM 710 (Zeiss). (B) Maximum intensity projection of z-stack & tile scan of a chip system on Day 2 of on-chip culture. Imaged on Cell Observer SD (Zeiss). Scale bar 200 µm.

### EXAMPLES

### 1. General

Here, the inventors disclose a microphysiological system (MPS) incorporating a microfluidic device, aiming to model a complex physiological microenvironment *in vitro.* By the way of example the inventors have established an *in-vivo-like* environment of pancreatic islets. The embodied endocrine pancreas-on-a-chip is a tailored microfluidic system, which enables self-guided immobilization of single spheroids at defined locations to enable *in situ* analysis on-chip. Pancreatic beta cells are assembled to 3-D cell clusters, so called pseudo-islets, and loaded to the system. They are further embedded within an ECM-like hydrogel emulating the physiological microenvironment, and allowing the integration of further tissue-relevant cell types; thereby improving culture conditions towards a microphysiological pancreas-on-chip platform.

The optical accessibility of the tissue on-chip provides the opportunity for non-invasive, real-time imaging to monitor viability and functionality. To track insulin secretion kinetics in response to glucose stimulation and metabolic activity on-chip in a time-resolved manner, dynamic sampling of the effluent as well as online, non-invasive real-time monitoring of the oxygen consumption, utilizing integrated optical oxygen sensors, were employed. Glucose treatment showed an increase in insulin secretion and oxygen consumption confirming cell functionality on-chip and higher metabolic activity of glucose-stimulated islets.

Pancreatic *in vitro* research is of major importance to advance mechanistic understanding, pharmaceutical research and treatment in the field of Diabetes Mellitus (DM). The disclosed MPS is a promising tool supporting 3-D tissue culture, e.g. modelling pancreatic islets, and the integration of further relevant tissue components, while providing an *in vitro* platform for non-invasive analysis of cell (patho-) physiology and response to drug treatment.

### 2. Material and methods

### Design and fabrication of the microfluidic device

In Fig. 1A, a schematic exploded view of an embodiment of the microfluidic device according to the invention is shown with the reference sign 10. The microfluidic device is of sandwich construction. It comprises a first microfluidic channel 12, a second microfluidic channel 14 and a porous membrane 16. The first microfluidic channel 12 is provided or embedded in a first plate 26 and the second microfluidic channel 14 is embedded in a second plate 28. In this regard, the first plate 26 and the second plate 28 are superimposed and aligned with the porous membrane 16 disposed there between such that the first microfluidic channel 12 and the second microfluidic channel 14 are essentially parallel to each other in the section s and are separated only by the porous membrane 16.

The first microfluidic channel 12 has an inlet opening 19 at a first end, and has a dead end structure 20 at a second end. The second microfluidic channel 14 has an inlet opening 22 at a first end and an outlet opening 24 at a second end beyond or downstream the common section s.

The first plate 26 has a bottom plate 30 adjacent its bottom surface, and the second plate 28 has a top plate 32 adjacent its top surface. The porous membrane 16, the second plate 28, and the top plate 32 each include through-holes 38, 40, 42 aligned with the inlet opening 19 of the first microfluidic channel 12. The top plate 32 also includes an opening 34 in the form of a through hole aligned with the inlet opening 22 in the second plate 28. Further, the top plate 32 further comprises another opening 36 in the form of a through hole aligned with the outlet opening 24 in the second plate 28.

As shown enlarged in Fig. 1B, the porous membrane 16 has so-called trapping structures, three of which are indicated with reference number 18, which are formed as through-holes.

The microfluidic device according to the invention 10 is also referred to as chip or microphysiological chip. All chip designs were executed using a computer-aided design software.

The microfluidic device 10 or the chip was fabricated by the inventors as a prototype using different materials, including PMMA (Plexiglas Resist, Evonik), PC (ip-CELLCULTURE^{™} track-etched membrane, it4ip) and PDMS (Sylgard 184, Dow Corning). In the simplest set-up, the microfluidic device 10 or chip comprises 250 µm thick PMMA media (= second plate 28) and tissue layers (= first plate 26), which entail the specific channel and chamber geometries and are separated by an approx. 22 µm thin porous PC membrane (= porous membrane 16) (Figure 1B). The media channel (= second microfluidic channel 14) and tissue chamber (= first microfluidic channel 12) both feature a width of approx. 500 µm, with a tissue channel (= first microfluidic channel 12) leading to the chamber being approx. 250 µm wide. The approx. 250 µm thick PMMA top layer (= second plate 28) is covered with an additional flexibel approx. 3 mm thick PDMS slab (= top plate 32) serving as an interface for tubing connections providing access to the in- (= inlet opening 19; through-holes 38, 40, 42) and outlets (= outlet opening 24; opening 36) of the microfluidic channel (= first and second microfluidic channels 12, 14) structures. The bottom layer (= bottom plate 30) is approx. 175 µm thin and allows for optical accessibility, while also serving as the sensor substrate in case of sensor integration to the system. In this case an approx. 250 µm wide line of oxygen indicator dye is spotted on the layer (= bottom plate 30) at the location of the tissue chamber. Any kind of contactless optical sensor can be integrated to the system using any of the chip layers as the sensor substrate.

PMMA layers (= first plate 26, second plate 28) and PC membranes (= porous membrane 16) were structured using a CO₂- or UV-lasercutter, respectively. The specific trapping structures (18) featured in the PC membrane (= porous membrane 16) were also generated by lasercutting approx. 50 µm holes. PDMS slabs (= bottom plate 30; top plate 32) for connection layers were fabricated by mixing PDMS prepolymer and curing agent in a 10:1 w/w ratio. To mold the slab 40 g of the uncured mix were poured into a squared petri dish and cured overnight at 60 °C. Afterwards the chip geometry was pre-structured into the PDMS using a lasercutter and cut out using a surgical knife. In- and outlets were punched using either a 0.35 mm or a 0.75 mm biopsy punch. The PDMS connection layer was bonded to the PMMA top layer using APTES functionalization and O₂-plasma activation.

PMMA layers (= first plate 26, second plate 28) were first aligned and then bonded at 125 to 130 °C in a preheated convection oven (Memmert) by placing the PMMA pieces (= first plate 26, second plate 28) between two microscopic glass slides and applying pressure from both sides using fold back clips. Chip (= microfluidic device 10) assembly was achieved in two consecutive bonding steps, for 15 min each: (i) first bonding membrane (= porous membrane 16) to the media channel (second microfluidic channel 14) and (ii) in the second step assembling the whole chip (= microfluidic device 10).

For GSIS experiments, fabricated PDMS wells (h = 3 mm, Ø = 4 mm) were bonded on top of the outlet (= opening 36) of the media channel (= second microfluidic channel 14) using O₂-plasma activation.

### Cell culture and pseudo-islet formation

The rat insulinoma-derived pancreatic beta cell line INS-1E was used in the experiments. Cells were cultured in T25 cell culture flasks (seeding density 40.000 cells/cm²) under standard conditions (37 °C, 5 % CO₂, 20 % O₂, 95 % humidity) and passaged at a confluency of 70-80 % using 0.05 % Trypsin/EDTA solution (Gibco). Culture media RPMI 1640 was supplemented with 10 mM Hepes buffer solution (Gibco), 1 mM sodium pyruvate (Gibco), 50 µM β-mercaptoethanol (Gibco), 5 % FCS (HyClone Fetal Clone II, GE Life Science), and 1 % penicillin-streptomycin (stock: 10.000 U/mL-10 mg/mL; Gibco).

Pseudo-islets were formed using 96 well ultra-low attachment (ULA) round bottom plates (Greiner Bio-One) at a concentration of 500 cells/well in 100 µL media/well as described in the art. Pseudo-islets were formed over 72 hours under standard cell culture conditions and then injected to the chip system.

### Tissue and hydrogel loading to the chip system & on-chip culture

Prior to cell injection, chips were oxygen plasma-treated for hydrophilization for 5 min. Subsequently, the chips were flushed with 70 % ethanol and then washed with PBS three times, leaving a pipet tip in the tissue chamber inlet and the in- and outlet of the media channel open.

Spheroids were grouped in one well of the ULA plate and then injected to the inlet pipet tip of the tissue chamber. Subsequently, the hydrogel (FibriCol, Advanced Biomatrix), optional with other embedded cell types, was loaded to the microfluidic chip through the pipet tip located in the inlet of the tissue channel. Chips are then placed under standard cell culture conditions to allow for hydrogel crosslinking. After a 60 min incubation, the pipet tip in the tissue inlet was removed and the tissue chamber closed off with PCR foil. The media channel of the chip was then connected via Tygon tubing (VERNAAAD04103, VWR international GmbH) to a 12-channel syringe pump (Landgraf Laborsysteme HLL GmbH) set-up and perfused with 20 µL/h applying positive pressure. Chips were cultured in an incubator at 37 °C, 5 % CO₂ atmosphere and 95 % humidity.

### Cell viability staining

Live-/dead staining was performed on-chip using fluorescein diacetate (FDA, Thermo Fisher) at 27 µg/mL visualizing living cells and propidium iodide (PI, Sigma Aldrich) at 135 µg/mL marking dead cells. Cell nuclei were stained using DAPI at 1 µg/mL (Thermo Fisher). On the respective day of viability assessment, chips were disconnected from the perfusion set-up and washed with PBS+ by gravity flow. Subsequently, a staining solution with FDA, PI & DAPI was prepared in PBS and injected to the media channel of the chip system by gravity flow and incubated for 15 min. Chips were washed with PBS-three times, and immediately imaged on a confocal microscope (Zeiss LSM 710 & Zeiss Axio Z.1 Cell Observer Spinning Disk).

### Immunofluorescence staining

To investigate tissue integrity, structure and function on-chip, stainings to visualize e-cadherin, insulin, f-actin and DAPI were applied.

All washing and staining solutions were flushed through the media channel by gravity flow. Before fixation, chips were disconnected from the pump set-up and flushed with PBS+. Fixation was performed by a 20 min incubation step with 4 % Roti^{®}Histofix (Carl Roth GmbH & Co. KG). After a thorough washing step with PBS- chips were stored at 4 °C until further processing. Blocking of unspecific binding and permeabilization was performed using 0.1 % Triton-X100 and 3 % normal donkey serum in PBS- for 1 h. Primary antibodies anti-insulin (1:200, ab181547, abcam) and anti-e-cadherin (1:50, BD610181, BD Bioscience) were diluted in antibody diluent (PBS- with final concentrations of 0.01 % Trition-X100 and 0.3 % normal donkey serum) and incubated for 2 h at RT and overnight at 4 °C. Chips were washed thoroughly by flushing the media channel three times with washing buffer (PBS- with 0.01 % Triton-X100 and 0.3 % normal donkey serum) and then incubated with secondary and conjugated antibodies as well as DAPI. Secondary antibodies Alexa Fluor 647 donkey anti-rabbit (1:100, A31573, Thermo Fisher) and Alexa Fluor 488 donkey anti-mouse (1:100, A21202, Thermo Fisher) as well as Alexa Fluor 546 Phalloidin (1:100, A22283, Thermo Fisher) and DAPI (1:1000, MBD0015, Merck KGaA) were diluted in antibody diluent and incubated for 2 h at RT and then thoroughly washed three times using washing buffer. Chips were stored in PBS- at 4 °C until imaging using the confocal microscope Axio Z.1 Cell Observer Spinning Disk (Carl Zeiss AG).

### Glucose-stimulated insulin secretion assay (GSIS) on-chip

Chips were loaded and cultured overnight (20 µL/h) as described above. To allow switching between different conditions the chips were connected to 4-port microfluidic valves. Prior to perfusion with low (3.3 mM in KREBS buffer) and high (16.7 mM in KREBS buffer) glucose conditions, a synchronization step was performed, perfusing the chips with KREBS 1x buffer containing 25 mM Hepes (Gibco), 0.1 % BSA (Sigma- Aldrich) and 0 mM glucose for 1 h. Subsequently, the chips were perfused with low, high and low glucose conditions for 1 h each. Effluent was sampled from the well on top of the outlet of the media channel every 60 min and stored at -20 °C until insulin analysis. Sampling time points were calculated based on volume of the chip, tubing and valves. Insulin secretion was quantified using Rat Insulin ELISA kit (Mercodia) following manufacturer's instructions.

### 3. Results

### Microfluidic chip concept and design

To generate a microphysiological platform modelling endocrine pancreas physiology and pathology, several key features of the in vivo microenvironment were considered. Here, the insulin secreting beta cells are part of 3D cell clusters, which are embedded in a unique microenvironment and highly vascularized. To this end the microfluidic platform is a tailored multiple-layered hybrid device featuring at least two channel geometries separated by a semi-permeable membrane (Figure 1A). The chip is fabricated using mainly PMMA exhibiting lower absorption of hydrophobic molecules compared to the predominantly used material of the field PDMS. The direct integration of trapping structures in the membrane (Figure 1B) enables the immobilization of cell spheroids at defined positions in the bottom channel of the chip allowing analysis by time-resolved read-out methods (illustrated in Figure 1C). The loading process enables self-guided trapping and thereby the integration of the spheroids on the system (Figure 1D).

The subsequent *in vitro* culture of the tissue under physiological conditions is realized by the chip set-up allowing the additional integration of an ECM-like hydrogel and co-culture with other relevant cell types of the tissue. Media supply is implemented through dynamic micro-sale fluid flow in the overlaying channel. The controlled fluid flow not only allows for stable nutrient supply and waste removal, but also enables dynamic sampling of the effluent to examine secretion kinetics in a time-resolved manner, such as glucose-stimulated insulin secretion, which is a key function of beta cells. Furthermore, the possibility to integrate contactless optical sensors, e.g. for oxygen measurements, to the system enables online, non-invasive real-time monitoring of the oxygen consumption of the cells directly on-chip. The geometries of the system can be adjusted to spheroids of different size and number. Depending on the objective and application of the system different materials can be used for its fabrication and the variable set-up allows the implementation of different kinds of port connection for controlled fluid flow on-chip.

### Tissue integration to the chip system

For chip development the previously established *in vitro* model mimicking insulin-secreting endocrine function of beta cells was employed. Pseudo-islets providing important physiological cell-cell contacts were formed by spontaneous aggregation of 500 cells/well to an average diameter of 150 µm over 72 hours using U-bottom ultra-low attachment wellplates.

Following the developed injection protocols based on hydrostatic pressure-driven flow resulting from the height difference of the tissue inlet compared to the outlets of the media channel the desired number of spheroids matching the trap number on-chip and ECM-like hydrogel can be loaded to the microfluidic system (Figure 1D). Using this loading principle spheroids are guided to the defined positions on-chip and the hydrogel retains the 3-D beta-cell-like microtissues in place during on-chip culture while at the same time mimicking the in vivo microenvironment of the cells. By the easy integration of other cell types to the hydrogel further tissue relevant components (e.g. endothelial cells) can be integrated. To investigate the homogenous loading of the hydrogel to the channel, fluorescent microspheres were integrated to the hydrogel before chip injection (Figure 1E). The maximum intensity projection of the z-stack acquired after overnight culture of the chip system confirms the homogenous filling of the whole tissue channel.

### Viability assessment on-chip

To investigate the impact of the loading process and on-chip culture on the viability of the tissue on-chip FDA/PI-based live/dead staining was performed, revealing predominantly viable cells on-chip with few scattered dead cells across the aggregates (Figure 2).

### Structural & functional characterization of the tissue on-chip

### - Insulin and e-cadherin expression on-chip

To assess pseudo-islet integrity and functionality on-chip e-cadherin and insulin expression were analyzed. E-cadherin is an important cell-cell contact protein and further influences the insulin secretion capacity of beta cells. Acquired confocal images demonstrate that e-cadherin as well as insulin remained highly expressed in the pseudo-islets on-chip and cells overall displayed intrinsic insulin production (Figure 3).

### - Glucose-stimulated insulin secretion on-chip

The key function of beta-cells is to secrete insulin in response to glucose. To analyze functionality on-chip glucose-stimulated insulin secretion (GSIS) assay was performed by sequentially incubating cells in low and high glucose conditions. Beta-cells on-chip demonstrated glucose responsiveness by an increased insulin secretion in response to high glucose stimulation, while declining again when exposed to low glucose levels (Figure 4).

### On-chip co-culture with tissue relevant cell types

Pancreatic islets are highly vascularized *in vivo* and there is a strong functional and physical interaction of beta and endothelial cells. Furthermore, endothelial cells support glucose sensing and endocrine hormone secretion highlighting the critical role of the close proximity of these cell types. The microfluidic chip set-up enables the integration of further cell types embedded in the hydrogel to the tissue chamber. Here, endothelial cells were suspended to a mixture of Collagen 1 and Fibrin loaded to the chip system surrounding the pseudo-islets. Successful loading and 3-D distribution of the cells visualized by CellTracker in the hydrogel could be confirmed (Figure 5A). Over a culture period of two days on-chip cells already started to assemble to 3-D vessel-like structures characterized by CD-31 immunofluorescent staining and pseudo-islets remained highly functional as confirmed by intrinsic insulin expression (Figure 5B).

## Claims

1. A microfluidic device (10), comprising a first microfluidic channel (12), a second microfluidic channel (14) and a porous membrane (16), wherein at least a section (s) of said first (12) and second microfluidic channels (14) is separated by said porous membrane (16), **characterized in that** said porous membrane (16) comprises trapping structures (18) at predefined positions configured to immobilize biological material.

2. The microfluidic device (10) of claim 1, **characterized in that** said trapping structures (18) are through holes in said porous membrane (16).

3. The microfluidic device (10) of claim 2, **characterized in that** said through holes comprise a mean diameter of approx. 30 - 70 µm, preferably approx. 40 - 60 µm, further preferably approx. 50 µm.

4. The microfluidic device (10) of any of the preceding claims, **characterized in that** said first microfluidic channel (12) comprises, at a first end, an inlet opening (19), and/or said first microfluidic channel (12) comprises, at a second end, a dead end structure (20).

5. The microfluidic device (10) of any of the preceding claims, **characterized in that** said second microfluidic channel (14) comprises, at a first end, an inlet opening (22) and/or, at a second end, an outlet opening (24).

6. The microfluidic device (10) of any of the preceding claims, **characterized in that** said first microfluidic channel (12) is provided in a first plate (26) and/or said second microfluidic channel (12) is provided in a second plate (28).

7. The microfluidic device (10) of any of the preceding claims, **characterized in that** said porous membrane (16) comprises pores comprising a mean diameter of approx. 1-10 µm, preferably approx. 1.5-8 µm, further preferably approx. 2-3 µm.

8. The microfluidic device (10) of any of the claims 8-13, comprising a bottom plate (30) adjacent to said first plate (26), and/or comprising a top plate (32) adjacent to said second plate (28).

9. The microfluidic device (10) of claim 8, **characterized in that** said top plate (32) comprises openings (34, 36) connected to said inlet opening (22) and/or said outlet opening (24).

10. The microfluidic device (10) of any of claims 6-9, **characterized in that** said first (26) and/or second (28) and/or bottom (30) and/or top plates (32) are transparent.

11. The microfluidic device (10) of any of claims 16-18, **characterized in that** said bottom plate (30) comprises a sensor, preferably an oxygen sensor.

12. A method of culturing biological material, comprising: a) providing the microfluidic device of any of claims 1-11; b) introducing biological material into said first microfluidic channel; c) introducing culture medium into said second microfluidic channel, and d) culturing said biological material under conditions allowing a physiological functioning of said biological material.

13. The method of claim 12, **characterized in that** said biological material comprises cellular aggregates, preferably cell spheroids, preferably comprising insulin-producing cells, further preferably beta cells.

14. The method of any of the preceding claims, **characterized in that** in step (b) further material is introduced into said first microfluidic channel preferably said further material comprises biological cells and/or hydrogel, preferably ECM-like hydrogel.

15. The method of any of the preceding claims, **characterized in that** said introduction in step (b) is realized via the application of a hydrostatic-pressure driven flow.
